Europäisches Patentamt

(19) European Patent Office

Office européen des brevets

(11) EP 0 872 173 A1

(12) EUROPEAN PATENT APPLICATION

(43) Date of publication:
21.10.1998 Bulletin 1998/43

(51) Int. Cl.$^6$: A01H 5/02

(21) Application number: 98106908.1

(22) Date of filing: 16.04.1998

(84) Designated Contracting States:
AT BE CH CY DE DK ES FI FR GB GR IE IT LI LU MC NL PT SE
Designated Extension States:
AL LT LV MK RO SI

(30) Priority: 16.04.1997 US 43953 P

(71) Applicant:
YISSUM RESEARCH DEVELOPMENT COMPANY OF THE HEBREW UNIVERSITY OF JERUSALEM
Jerusalem 91042 (IL)

(72) Inventors:
• Basnizki, Yehuda
  Moshav Beit Nekofa 90830 (IL)
• Zohari, Daniel
  Jerusalem 93704 (IL)

(74) Representative:
Modiano, Guido, Dr.-Ing. et al
Modiano, Josif, Pisanty & Staub,
Baaderstrasse 3
80469 München (DE)

(54) Hybrid seeds of globe artichoke for seed planting and method of producing same

(57) Globe artichoke plants, heads and seeds containing a recessive male sterility mutant allele in a heterozygous or homozygous form, which allele is non-allelic with $ms_1$, as tested in a test for allelism. These plants, when in a homozygous form may be used to produce hybrid seeds for commercial globe artichoke seed planting when crossed with a pollen donor having a normal, fertile phenotype. Further described is a method of producing such hybrid seeds.

Fig. 3b

EP 0 872 173 A1

**Description**

FIELD AND BACKGROUND OF THE INVENTION

The present invention relates to new and distinct globe artichoke *(Cynara scolymus* L.) plants, heads and seeds and, more particularly, to artichoke plants which are homozygous for either one of two new non-allelic male sterility recessive genes. The present invention further relates to hybrid seeds produced by such plants when pollinated with pollen produced by a male fertile globe artichoke plant and to hybrid plants and heads resulting front these seeds. Most particularly the present invention relates to artichoke plants, heads and seeds which carry at least one recessive male sterility gene, hereinafter referred to as $ms_2$ and $ms_3$ in a homozygous or heterozygous form. Yet, the invention also relates to a method of producing hybrid globe artichoke seeds for commercial seedplanting of globe artichoke.

Male sterility in plants implies an inability to produce or to release functional (fertile) pollen. Male sterility in plants results in failure of formation or development of functional stamens, microspores or gametes.

Increasing interest in both breeding of hybrid varieties and efficient methods of population improvement has made pollination control via male sterility an important objective in many plant breeding programs.

From a structural/functional point of view, male sterility in plants may be divided into three categories which include (i) pollen sterility, Wherein functional pollen grains are missing: (ii) structural (or staminal) male sterility, wherein male flowers or stamens are malformed and therefore non-functional, or missing altogether; and (iii) functional male sterility, wherein good and viable pollen is trapped in indehiscent anthers and thus prevented from functioning.

From a genetic point of view, male sterility in plants may also be divided into three categories which include (i) nuclear male sterility (NMS). also known in the art as genic or Mendelian male sterility, wherein male sterility is governed solely by one or more nuclear genes: (ii) cytoplasmic male sterility (CMS), wherein male sterility results due to a combined action of nuclear and cytoplasmic organelle (e.g.. mitochondria or chloroplasts) genes; and (iii) non-genetic male sterility which is either chemically or mechanically (pollen removal) induced.

Nuclear male sterility is a common phenomenon in nature, resulting from spontaneous or induced mutations. In most cases studied, spontaneous nuclear male sterility is controlled by a single recessive gene designated *ms,* as opposed to *Ms* or +, which confers normal production of pollen.

Male sterility plays an important role in plant breeding (i) in the production of hybrid seeds for hybrid seed planting: and (ii) as a plant breeding tool facilitating population improvement, backcrossing, testcrossing for combining ability, interspecific and intergeneric hybridization and other intermediate breeding procedures.

Hybrid breeding methods are successfully employed in various vegetables and ornamental crops. An effective hybrid breeding method permits the production of hybrid seeds in a quantity such that the F1 generation can be grown directly by the farmer. This is not much of a problem in cases where the male flowers can be easily removed without damaging the female flowers. However, such anatomy is the characteristic of a limited number of plant species (e.g., corn), hence plant breeders used different kinds of male sterility to circumvent the restrictions to large scale controlled hybridization imposed by flower morphology and breeding systems.

The fact that nuclear male sterility (NMS) does not permit the production of a uniformly male sterile population seriously limits its use in hybrid seed production. To permit the use of recessive NMS in large scale commercial hybrid seed production, it is necessary to rogue out 50% male fertile plants from the backcross *msms* x *Msms*. Since safe classification of fertile versus male sterile plants cannot usually be made until shortly before the stage of anthesis (i.e., anther maturation) during flowering, such roguing becomes far too difficult and costly' in most crops to permit economic production of large quantities of hybrid seeds.

Several solutions to this limitation have been exercised, including (i) \vegetative propagation, as hereinbelow further described; (ii) marker assisted selection, e.g., a genetically linked color gene which is detected early after germination, much before flowering; (iii) cytogenetic methods: (iv) temporary restoration of fertility under selective environmental conditions; and (v) use of functional male sterility. Further details concerning these options are found in J. M. Lasa and N. O. Bosemark (1993) Male sterility. In M. D. Hayward. N. O. Bosemark: and I. Romagosa Eds. "Plant breeding principles and prospects" Cha. 14. pp. 213-228 and in the references therein cited, all are incorporated by reference as if fully set forth herein.

Vegetative propagation of male sterile plants to be used as females (seed bearers) in hybrid seed production has so far been used mainly in ornamental plants, where the price of commercial seed is high. However. through the development of cost effective efficient micropropagation techniques, hybrid seed production based on vegetatively propagated homozygous male sterile genotypes is likely to increase in importance. especially among vegetable crops.

Globe artichoke *(Cynara scolymus* L.) is a large thistle of the daisy family. Compositae (Asteraceae), native to the Mediterranean basin. It is a robust. perennial diploid (2n = 2x = 34 ), predominantly a cross-pollinated vegetable, with a characteristic rosette of big leaves, grown for its large. fleshy heads. Commercially, globe artichoke is traditionally grown by vegetative propagation of suckers.

However, the traditional cultivation of globe artichoke by vegetative propagation is a costly operation involving considerable labor input. Because of heavy investments in establishment, growers maintain their plantations for several years, even though yields and quality frequently decrease considerably in subsequent years. Perennial. Vegetatively propagated plantations also incur heavy risks of damage from diseases and pests. The development of meristem-derived plantlets did not alleviate many of these difficulties. Moreover, the cost of biotechnologically produced plants is prohibitively high and field planting is labor intensive.

On the other hand, seed-planted cultivars enjoy many advantages. The major advantages of seed-planted cultivars are as follows.

First, compared to planting of suckers or meristem-produced plants. mechanical seed sowing is a labor saving and therefore a cheaper operation.

Second, in a Mediterranean climate, and if sown early enough in the fall, most artichoke genotypes bolt fully the following spring. Thus, by shifting to seed-planting, this perennial plant can be converted into an annually grown crop, which may therefore be introduced into crop rotation. The time span from seeding to the end of the harvest is seven to eight month.

Third, direct seeded plants develop long vertical taproots, which penetrate deeper into the soil than the adventitious roots produced by planted suckers. Therefore, compared to vegetatively propagated plants. they utilize moisture and fertilizers more efficiently.

And finally, for pest and disease control, the shift to seed-planting prevents the transfer and spread of soil-borne and other pathogens (such as bacteria and viruses) and pests. Such contaminations are comnlon when \vegetative propagation is implemented to start new plantations. Seedplanting also prevents virus infections, because most plant viruses are not transmissible via seeds. Thus, annual cultivation, coupled with crop rotation. protects the grower from the ravages of pests and epidemics much better than perennial planting.

All these elements contribute to more vigorous plant growth. healthier plants, and lower input of pesticides; fungicides and fertilizers. In other words, seed-planting makes globe artichoke more friendly both to the grower and to the environment.

However, due to the genetic heterogeneity and high level of heterozygosity characterizing each of the known artichoke cultivars. selflng of clonal artichoke cultivars results in wide morphological segregation. Furthermore, repeated selfing results in a considerable inbreeding depression. In some cases the effects of inbreeding are so severe that it is impossible to continue selfing beyond three or four generations, wherein depression effects are observable as early as the second selfing generation. For further detail. see Y. Basnizki and D. Zohary (1994) Breeding of seed planted artichoke. In. J. Janik ed. Plant Breeding Reviews. John Wiley and Sons. Inc., which is incorporated by reference as if fully set forth herein. As hereinabove discussed, male sterility is an excellent tool to prevent selfing.

To date, only genic (Mendelian) male sterility has been detected in artichoke. Principe (J. A. Principe (1984) Male sterility in artichoke. HortScience 19:864-865, which is incorporated by reference as if fully set forth herein) reported on male sterility govemed by a single recessive gene. which was detected in an unidentified globe artichoke material grown in California, and which is herein designated $ms_1$. However, the $ms_1$ gene was not used in hybrid seed production for commercial seed-planting of globe artichoke. Furthermore, the $ms_1$ gene is so far known only in the context of Principe's Californian globe artichoke, which, as of yet, was not employed to produce a commercial seed planted variety.

There is thus a widely recognized need for, and it would be highly advantageous to have, artichoke plants which are homozygous for either one of two new non-allelic male sterility genes, which could be used as female parents for production of seed-planted hybrid artichoke varieties of selected properties.

SUMMARY OF THE INVENTION

According to the present invention there are provided globe artichoke plants, heads and seeds and a method of producing hybrid artichoke seeds for commercial seed-planting of globe artichoke.

According to further features in preferred embodiments of the invention described below, provided is an artichoke plant containing a recessive male sterility mutant allele in a heterozygous and homozygous form. The allele being non-allelic with $ms_1$. The term $ms_x$ is herein introduced for designating any nuclear male sterility gene which is non allelic with $ms_1$.

According to still further features in the described preferred embodiments the allele is a result of a spontaneous mutation.

According to still further features in the described preferred embodiments the allele, when in the homozygous form, yields plants having largely shriveled, almost fully aborted pollen grains.

According to still further features in the described preferred embodiments the allele, when in the homozygous form, yields pollenless plants.

According to still further features in the described preferred embodiments the allele is $ms_2$, or $ms_3$.

According to still further features in the described preferred embodiments provided is an artichoke head produced by any of the plants hereinabove described.

According to still further features in the described preferred embodiments the head mimics the phenotype of a vegetatively propagated commercial artichoke variety (as for example listed in Table 1 below).

According to still further features in the described preferred embodiments the head mimics the phenotype of a vegetatively propagated commercial artichoke variety.

According to still further features in the described preferred embodiments provided is an artichoke seed containing a recessive male sterility mutant allele in a heterozygous or homozygous form, the allele being non-allele with $ms_1$.

According to still further features in the described preferred embodiments the allele is a result of a spontaneous mutation.

According to still further features in the described preferred embodiments the allele, when in the homozygous form. Yields plants having largely shriveled, almost fully aborted pollen grains.

According to still further features in the described preferred embodiments the allele, when in the homozygous form, yields plants having sterile, pollenless anthers.

According to still further features in the described preferred embodiments the allele is $ms_2$ or $ms_3$.

According to still further features in the described preferred embodiments provided is an artichoke plant male parent containing a recessive male sterility mutant allele in a homozygous form, the allele being $ms_2$ or $ms_3$.

According to still further features in the described preferred embodirnents provided is a heterozygous artichoke seed for commercial seed-planting resulting from crossing a female parent containing the $ms_2$ or $ms_3$ gene in a homozygous form with a male parent, the seed having a $ms_2$ or $ms_3$ gene as a heterozygote, respectively.

According to still further features in the described preferred embodiments provided is a heterozygous artichoke plant resulting from germinating any of the above seeds.

According to still further features in the described preferred enlbodinlents provided is a method of producing globe artichoke seeds for commercial seed-planting, the method comprising the steps of (a) crossing a female parent artichoke plant containing a recessive male sterility gene in a homozygous form with a male parent artichoke plant: and (b) collecting seeds developing on the female parent.

According to still further features in the described preferred embodiments of the method. The gene is selected from the group consisting of $ms_1$, $ms_2$ and $ms_3$ .

According to still further features in the described preferred embodiments of the method, an artichoke head derived from a plant produced from the seed mimics the phenotype of a vegetatively propagated commercial artichoke variety.

The present invention successfully addresses the shortcomings of the presently known configurations by providing globe artichoke male sterile plants for the production of artichoke seeds for commercial seed-planting for the production of globe artichoke plants with desired properties, which enjoy the advantages of seed-planting, as listed in the Background section above.

BRIEF DESCRIPTION OF THE DRAWINGS

The invention herein described, by way of example only, with reference to the accompanying drawings, wherein:

FIGs. 1a and 1b are photographs of an artichoke plant and a head of an artichoke plant of the "Cavo" line, respectively;

FIGs. 2a and 2b are schematic depictions of pollen grains produced by a $ms_2\ ms_2$ artichoke plant according to the present invention and of norilial pollen grains, respectively, wherein c indicates cytoplasm. e indicates the exine and p indicates the germination pores (ca. x 200):

FIGs. 3a and 3b are photographs of an artichoke plant and a head of an artichoke plant of the "Tudella" stock . respectively:

FIGs. 4a and 4b are schematic depictions of pollenless anther tubes and traverse sections of a $ms_3ms_3$ artichoke plant according to the present invention, and of pollen containing anthers of a normal artichoke plant, respectively;

FIG. 5 is a photograph of a hybrid variety globe artichoke plant # 223, heterozygous for the $ms_2$ allele, which phenotypically mimic the vegetatively propagated "Empolese" variety: and

FIG. 6 is a photograph of a hybrid variety globe artichoke plant # 494, heterozygous for the $ms_3$ allele, which phenotypically mimic the vegetatively propagated "Violetto de Toscana" variety.

DESCRIPTION OF THE PREFERRED EMBODIMENTS

The present invention relates to globe artichoke (*Cynara scolymus* L.) plants, heads and seeds which carry at least one male sterility recessive gene in a homozygous or heterozygous form. The novel characteristic of the artichoke plants, heads and seeds according to the present invention is that they include at least one recessive allele of one of two novel nuclear male sterility genes, herein referred to as $ms_2$ and $ms_3$. The present invention can be used to provide an agricultural tool for artichoke hybrid seed plantations devoid of selfed seeds and therefore devoid of phenotypic segregation and inbreeding depression. Specifically, the present invention can be used to provide vegetatively propagated $ms_2ms_2$ and/or $ms_3ms_3$ artichoke plants, which can be pollinated by normal pollen produced by normal male fertile artichoke plants to produce hybrid seeds of desired properties for commercial globe artichoke seed-planting.

Two new recessive male sterility mutations, designated HU-$ms_2$ and HU-$ms_3$ (HU for Hebrew University), referred to herein as $ms_2$ and $ms_3$. were discovered in globe artichoke and can be used in the development of seed production of seed planted hybrid varieties in this vegetable.

These two mutations were found to be non-allelic with one another. Both are also non-allelic with an additional prior art male sterility recessive mutation found by J.A. Principe (1984) in California, referred to herein as $ms_l$, all as herein further described.

These are spontaneous mutations discovered by selfing vegetatively propagated artichoke plants. The rational for searching male sterility mutations by selling clonal plants is that during the long history of vegetative propagation such clones accumulate spontaneous recessive mutations which are not detectable phenotypically since they are covered by a heterozygous dominant genetic background. Nevertheless, upon selfing. such mutations are discovered when two recessive male sterility alleles reside in a single individual plant. The selection for naturally occurring (i .e., spontaneous) mutations is presently preferred since when induced mutagenesis is of choice (e.g.. by chemical treatment or irradiation) various other genes become mutated in an uncontrolled and some times undesired fashion.

It should be noted that the terms 'mutation' 'mutant'. 'allele' and 'gene' are interchangeably used herein with respect to male sterility, with otherwise identical meaning; as these terms are frequently substitued for one another in the art of genetics.

The search for male sterility mutants according to the present invention was conducted in a collection (gene-pool) of traditional vegetatively propagated cultivars of globe artichoke (*Cynara scolymus* L.) assembled by J. Basnizki and D. Zohary at the Hebrew University of Jerusalem. Some of these. among them those which are most common and of commercial value, are listed in Table 1, below.

These and additional vegetatively propagated globe artichoke varieties are described in more detail, and accompanied by photographs, in Dellacecca. V., Magnifico, V., Marzi, V., Porceddu E. and Scaracia. G. T. (1967) Contributo alla conoscenza delle varieta di carciofo coltivat nel mondo. Atti 2° congres. Int. di Studi sul Carciofo, Bari. especially in pages 199-316 , which is incorporated by reference as if fully set forth herein.

Samples of seeds obtained from the various cultivars by selfing for few generation (e.g., 2-5) were planted, and the progenies raised were screened for individuals showing pollen abortion or sterile anthers.

When such a male sterile individual was detected, it was subjected to the following genetic tests.

First, it was cross-pollinated by pollen obtained from a true breeding. fully fertile pollen donor line to find out whether it was female fertile. i.e., that after such cross-pollination it produced seeds; and that these seeds germinated normally.

## TABLE 1

| | Type* (Country) | Start of harvest | Mean head weigh (grams) | Shape of head | Head height (cm) | Head diameter (cm) | Head color | Spininess of bracts | Heads/ plant | Heads/ plant** |
|---|---|---|---|---|---|---|---|---|---|---|
| 1 | Catanese (Italy) | Fall | 130-190 | Cylindric- conical | 10.5 | 7.0 | Green. tinged with violet | Spineless | 8 | 7 |
| 2 | Masedu (Italy) | Fall | 150-200 | Cylindrical | 10.5 | 7.5 | Green. tinged with violet | Spineless | 8 | 7 |
| 3 | Spinoso Sardo (Italy) | Fall | 160-220 | Conical | 13.5 | 7.5 | Green. tinged with violet- brown | Spiny | 10 | 3 |
| 4 | Spinoso di Palermo (Italy) | Fall | 200-260 | Ovoid | 12.0 | 8.0 | Green. tinged slightly with violet | Spiny | 7 | 3 |
| 5 | Violetto di Toscana (Italy) | Spring | 200-280 | Elliptical | 11.5 | 7.8 | Violet | Spineless (but mucronate) | 11 | 8 |
| 6 | Romanesco (Italy) | Spring | 300-450 | Round | 10.5 | 10.5 | Violet, tinged with green | Spineless | 8 | 7 |
| 7 | Empolese (Italy) | Spring | 250-300 | Round | 11.0 | 10.5 | Green. tinged with violet | Spineless | 8 | 7 |
| 8 | Molese (Italy) | Fall | 130-200 | Cylindrical | 10.5 | 7.5 | Green. tinged with violet | Spineless | 7 | 6 |
| 9 | Precoce de Iesi (Italy) | Spring | 250-320 | Ovoid | 11.5 | 9.3 | Violet, tinged with green | Spineless | 7 | 2 |
| 10 | Violet de Provence (France) | Fall | 180-240 | Ovoid | 11.0 | 7.5 | Violet, tinged with green | Spineless | 8 | 4 |
| 11 | Gros Camus de Bretagne (France) | Spring | 300-400 | Subspherical | 9.5 | 10.0 | Yellowish green | Spineless | 5 | 3 |
| 12 | Tudella (Spain) | Fall- Spring | 150-200 | Ovoid | 10.0 | 7.5 | Yellowish green | Spineless | 5 | 3 |
| 13 | Bianca de Espania (Spain)*** | Fall- Spring | 150-200 | Ovoid | 10.0 | 7.8 | Greenish | Spineless | 6 | 3 |
| 14 | Green Globe (USA) | Spring | 250-300 | Subspherical | 8.5 | 10.0 | Yellowish green | Spineless | 5 | 3 |

\*      The term "type" as used herein refers both to a clonal variety and a clonal stock.

\*\*     For 'sot olio' -- Small heads for processing with olive oil.

\*\*\*    In fact, "Bianca de Espania" is a part of the "Tudella" stock, which includes several clones which are slightly phenotypically distinct from one another.

And second, after female fertility was confirmed, the $F_1$ plants obtained from the above cross were further used to produce (i) $F_2$ generation by selfing: and (ii) $BC_1$ generation by crossing the $F_1$ hybrids back to their male sterile parent. A 3:1 pollen fertile versus pollen sterile segregation in the $F_2$ generation; and 1:1 segregation in the $BC_1$ generation. led to the conclusion that a detected male sterile individual was homozygous for a single male sterility recessive mutation. Two new male sterility non-allelic recessive mutations were discovered by these tests.

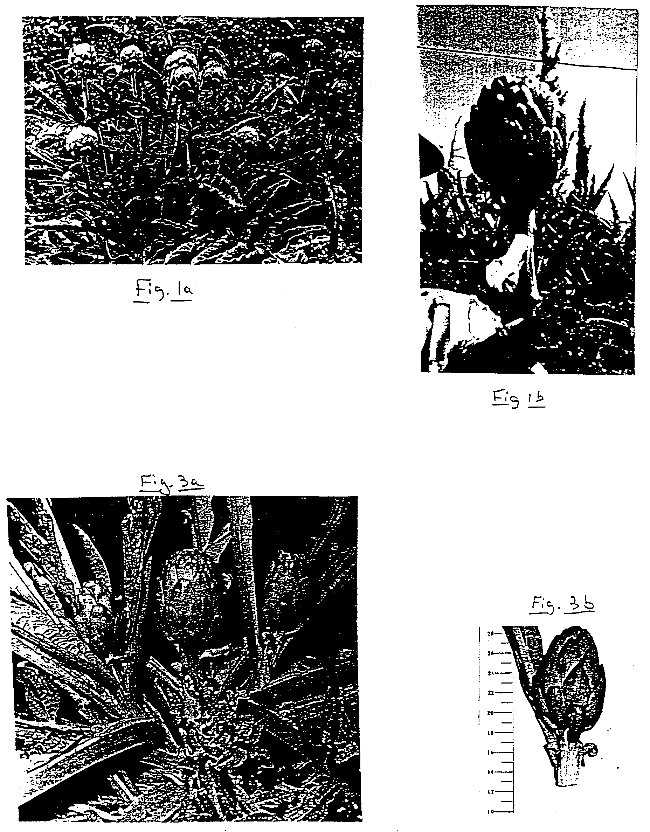

The first. designated $ms_2$, was extracted from the "Cavo" line after four generations of selfing and was given the HU accession # 072. A plant and a head of the "Cavo" line are shown in Figures 1a and 1b, respectively. The "Cavo" line was originated by crossing between Violet de Provence and Gros Camus de Bretange in France (see Table 1 for detail).

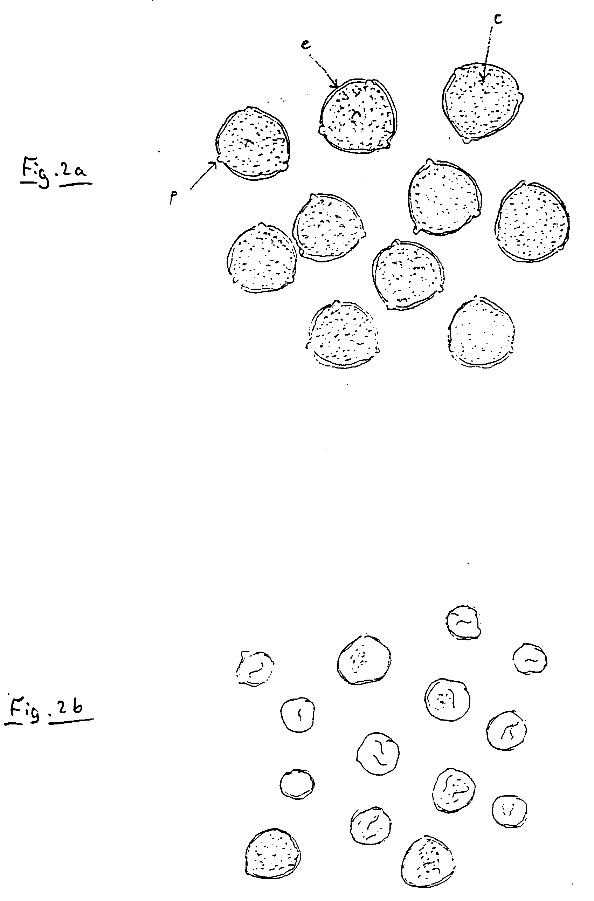

As shown in Figure 2a, mutants homozygous for this recessive gene are characterized by largely shriveled, almost fully aborted pollen grains, as compared with the normal pollen grains phenotype of the "Cavo" line. shown in Figure 2b.

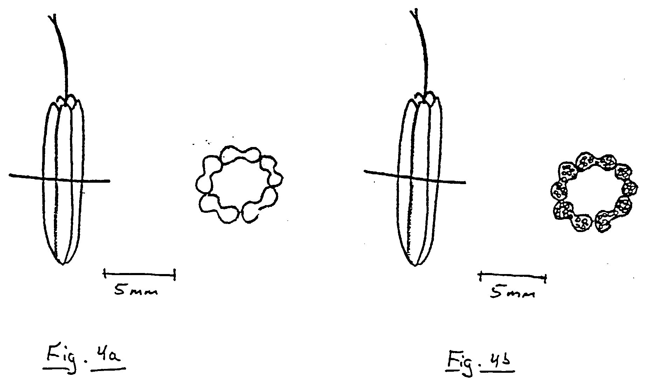

The second, designated $ms_3$ was extracted from the "Bianca de Espania", which is a part of the "Tudella" stock (see Table 1 for more detail), after three generations of selfing, and was given the HU accession # 113/3/3. A plant and a head of the "Tudella" stock are shown in Figures 3a and 3b. respectively.

As shown in Figure 4a, plants homozygous for the $ms_3$ recessive mutation are characterized by totally sterile (pollen-less) anthers. as compared to the pollen containing anthers of norinal artichoke plants of the "Tudella" stock. shown in Figure 4b.

The two newly discovered male sterility mutants $ms_2$ and $ms_3$, as well as the $ms_l$ recessive male sterility mutation found by Principe (J. A. Principe (1984) Male sterility in artichoke. HortScience 19:864-865) were subjected to tests for allelism, to establish whether these mutations were allelic (i.e., in the same gene) or non-allelic (i.e. mutations in different genes).

Because the mutant individuals were male sterile, the classical test for allelism, namely a direct cross between mutants (each homozygous for a recessive mutation) was not possible. Instead, the tests were carried out by crossing a male sterile homozygous recessive individual for one mutation to a pollen fertile plant heterozygous for another mutation: and examination of the progeny produced by such cross combination.

If two tested mutations are allelic, such a cross is expected to produce 50% male Fertile and 50% male sterile progeny. On the other hand. if the two tested mutations are non-allelic, the cross is expected to produce 100% male fertile progeny.

The following Hebrew University accessions were involved in the crosses:

(i) Male sterile homozygous mutants:

# 072 -- a male sterility mutant having a $ms_2ms_2$ genotype.
# 113/3/3 -- a male sterility mutant having a $ms_3 ms_3$ genotype.
# 155/8 -- a male sterility mutant having a $ms_1ms_1$ genotype.

(ii) Male parents for the F$_1$ hybrids:

# 201/4/2B -- a true breeding pollen donor line. which is a product of four generations of selfing of the "Violet de Provence" variety' (see Table 1 for detail), having a $ms_2Ms_2/Ms_3Ms_3$ genotype.

# 136/7 -- a true breeding pollen donor line; which is a product of four generations of selfing of the "Tudella" stock (see Table 1 for detail), having a $ms_2Ms_2/Ms_3Ms_3$ genotype.

Two male parents # 201/4/2B and # 136/7 were selected for testing due to different time of flowering.

(iii) Pollen fertile F$_1$ hybrids heterozygous for a male sterility mutation:

F$_1$ (072 x 201/4/2B) -- heterozygous for male sterility having a $Ms_2ms_2$ genotype.

F$_1$ (113/3/3 x 201/4/2B) -- heterozygous for male sterility having a $Ms_3ms_3$ genotype.

F$_1$ (113/3/3 x 136/7) -- heterozygous for male sterility having a $Ms_3ms_3$ genotype.

The results of the tests for allelism among the three male sterile mutants are given in Table 2, below.

TABLE 2

| Cross combination | Progenies examined | | |
|---|---|---|---|
| | male fertile | male sterile | total number |
| 113/3/3 x F$_1$ (072 x 201/4/2B) | 36 | - | 36 |
| 072 x F$_1$ (113/3/3 x 136/7) | 37 | - | 37 |
| 155/8 x F$_1$ (072 x 201/4/2B) | 35 | - | 35 |

TABLE 2 (continued)

| Cross combination | Progenies examined | | |
|---|---|---|---|
| | male fertile | male sterile | total number |
| 155/8 x F$_1$ (113/3/3 x 136/7) | 30 | - | 30 |

These results clearly demonstrate that no allelic relations exist among any of the $ms_1$, $ms_2$ and $ms_3$ mutations. These results are described in more detail hereinbelow.

Testing for allelism between the $ms_2$ and $ms_3$ mutations.

The homozygous recessive male sterile mutant # 113/3/3 ($ms_3ms_3$) was crossed with an F$_1$ hybrid obtained by crossing the homozygous recessive male sterile mutant # 072 ($ms_2ms_2$) with pollen donor line # 201/4/2B. 36 progenies were obtained. All were pollen fertile. This result establish that the two new mutations $ms_2$ and $ms_3$ are non allelic.

Testing for allelism between the $ms_2$ or the $ms_3$ mutations and the $ms_1$ mutation reported in 1984 by Principe.

Seeds produced by selfing of a heterozygous plant carrying the californian male sterile mutant $ms_1$ were kindly provided by Dr. J. A. Principe. These seeds were grown in order to detect segregating homazygous mutant plants. designated HU # 158/8. see Table 2. above. This mutant showed almost fully aborted pollen. The same crossing design was used to find out the allelic relationships between the two male sterile genes $ms_2$, and $ms_3$ herein disclosed and the recessive male sterility mutant $ms_1$ reported by Principe. Principe's homozygous recessive male sterile mutant plants (HU # 155/8) were crossed both with F$_1$ hybrids heterozygous for $ms_2$ or $ms_3$ (see Table 2 above). All the progenies obtained from these crosses were male fertile, showing that the recessive gene discovered by Dr. Principe is non-allelic with respect to the new male sterility $ms_2$ and $ms_3$ genes.

Confirmation tests.

To forther confirm a Mendelian allelic segregation among plants carrying a given male sterility mutation. crosses were also independently made (i) between $Ms_2ms_2$ heterozygote derivatives of the "Cavo" line and $ms_2ms_2$ homozygote mutants and (ii) between $Ms_3ms_3$ heterozygate derivatives of the "Tudella" stock and $ms_2ms_2$ homozygote mutants. As expected in such allelic crosses. the progeny obtained were ca. 50% male fertile and ca. 50% male sterile.

Reference in now made to the following example, which together with the above descriptions, illustrate the invention.

**EXAMPLE**

Globe artichoke hybrid varieties which phenotypically mimic vegetatively propagated commercial varieties and which are heterozygous for either the $ms_2$ or $ms_3$ alleles were selected by examining the phenotypes of several hundreds cross combinations in which male sterile females of the genotype $ms_2ms_2$ or $ms_3ms_3$ were pollinated with pollen produced by normal males of the genotype $Ms_2Ms_2/Ms_3Ms_3$. These parents were obtained by several generations of self pollination and selection of globe artichoke plants of the vegetatively propagated varieties listed in Table 1 above. Two Examples of hybrid varieties thus obtained. a description of their parents and the vegetatively propagated variety which they mimic are found in Table 3 below.

TABLE 3

| | female parent | male parent | hybrid variety | mimic phenotype |
|---|---|---|---|---|
| 1 | # 072 | # 144 | #223 * | "Empolese" |
| 2 | # 113/3/146 | # 153/3B | # 494 ** | "Violetto di Toscana" |

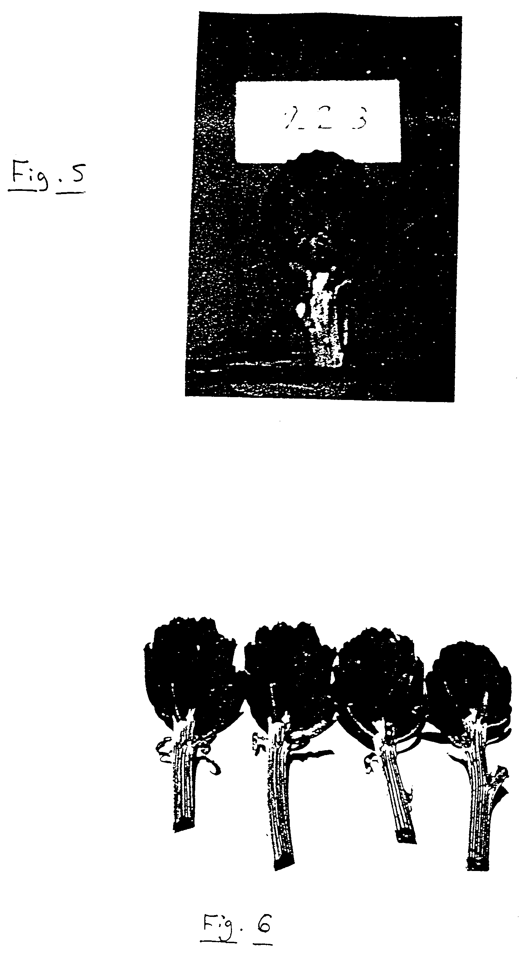

\* see Figure 5.
\*\* see Figure 6.

The # 072 is a female parent clone which was obtained by four generations of' selfing of the original "Cavo" line and selection for derivatives exhibiting $ms_2$ type male sterility combined with pronounced vegetative growth and full seed production when crossed with a male fertile pollen donor.

The # 144 is a male pollen donor parent clone which was obtained by three generations of selfing of the "JaJa" line, itself developed in France from the "Precoce de Iesi" variety, and selection for derivatives exhibiting pronounced vegetative growth, abundant normal pollen production and specific combining ability to generate a hybrid plant which mimics the "Empolese" variety. when crossed with # 072. The "JaJa" line was used in parallel to generate the "Talpiot" true breeding seed planted globe artichoke variety. developed and commercially available in Israel, by four generations of selfing and selection.

The # 113/3/146 is a female parent clone which was obtained by three generations of selfing of the original "Bianca de Espania" variety and selection for derivatives exhibiting $ms_3$ type male sterility combined with pronounced vegetative `growth and full seed production when crossed with a male fertile pollen donor.

The # 153/3B is a male pollen donor parent clone which was obtained by' three generations of selfing of the original "Molese" \variety and selection for derivatives exhibiting pronounced vegetative growth. abundant normal pollen production and specific combining ability to generate a hybrid plant that mimics the "Violetto di Toscana" variety, when crossed with # 113/3/146.

It should be noted that when the term 'mimics' is used herein in the speciflcation and especially in the claims section below, it refers to a situation where the phenotypic appearance of a specific head resembles that of a known vegetatively propagated variety to a degree which renders the market indifferent to whatever differences exist therebetween. Typically, a phenotypic appearance which more or less follows ally of those listed in Table 1 above will be indifferently accepted in the relevant markets although it is not derived from any of the varieties thereat listed, since it is a hybrid.

At present, the $ms_3$ allele is most preferred for commercial hybrid deed production for seed-planting of globe artichoke since it confers complete sterile. pollenless anthers when in a homozygous form. As such, a plant homozygote for the $ms_3$ allele cannot self under no circumstances. It should be appreciated that most recessive male sterility genes so far reported for any flowering plant do not confer such absolute male sterility.

As mentioned above, the $ms_2$ allele is associated with largely shriveled; almost fully aborted pollen grains. As such, these grains, under some conditions, are fertile and may therefore self to a limited extent.

Thus, according to the present invention provided are globe artichoke plants, heads and seeds containing a recessive male sterility allele in a heterozygous or homozygous form, which allele is non-allele with $ms_1$. as tested in a test for allelism. These plants. when in a homozygous form are preferably vegetatively propagated to provide male sterile plants. These plants may be crossed with a pollen donor (male) having a normal pollen phenotype (either heterozygous or preferably homozygous) to yield male sterility heterozygous seeds. These hybrid seeds are thereafter used for globe artichoke seed-planting to produce a hybrid variety to obtain a crop of artichoke heads of desired properties (i.e., which mimics the phenotype of a desired commercial artichoke variety, see, for examples. Table 1 above), yet to enjoy the advantages of the seed-planting methodology, as delineated in the Background section above.

Further according to the present invention provided is a method of producing globe artichoke seeds for commercial seed-planting, the method includes the steps of (a) crossing a female parent artichoke plant containing a recessive male sterility gene in a homozygous form with a male parent artichoke plant and (b) collecting seeds developing on the female parent.

It should be noted with this respect that. although the $ms_1$ gene is known for quite a while. it was so far not used for producing globe artichoke hybrid seeds for commercial seed-planting. Thus, the method according to the present invention is not intended to be limited to any specific male sterility gene and is therefore directed at any recessives male sterility gene including but not limited to $ms_1$ $ms_2$ and $ms_3$.

In a preferred embodiment of the invention the male and female parents are selected such that the artichoke heads derived from a plant produced from the hybrid seeds mimic the phenotype of a vegetatively propagated commercial artichoke variety, selected for example from the varieties listed in Table 1, above.

While the invention has been described with respect to a limited number of embodiments, it will be appreciated that many variations. modifications and other applications of the invention may be made.

**Claims**

1. An artichoke plant containing a recessive male sterility mutant allele in a form selected from the group consisting of heterozygous and homozygous, said allele being non-allelic with $ms_1$.

2. The artichoke plant of claim 1. wherein said allele is a result of a spontaneous mutation.

3. The artichoke plant of claim 1, wherein said allele, when in laid homozygous form, yields plants having largely shriveled, almost fully aborted pollen grains.

4. The artichoke plant of claim 1, wherein said allele, when in said homozygous form, yields pollenless plants.

5. The artichoke plant of claim 1, wherein said allele is selected from the group consisting of $ms_2$ and $ms_3$.

6. An artichoke head produced by the plant of claim 1.

7. An artichoke head produced by the plant of claim 5.

8. The artichoke head of claim 6, wherein said head mimics a phenotype of a vegetatively propagated commercial artichoke variety.

9. The artichoke head of claim 7, wherein said head mimics a phenotype of a vegetatively propagated commercial artichoke variety.

10. An artichoke seed containing a recessive male sterility mutant allele in a form selected from the group consisting of heterozygous and homozygous, said allele being non-allelic with $ms_1$.

11. The artichoke seed of claim 10, wherein said allele is a result of a spontaneous mutation.

12. The artichoke seed of claim 10, wherein said allele, when in said homozygous form, yields plants having largely shriveled, almost fully aborted pollen grains.

13. The artichoke seed of claim 10, wherein said allele, when in laid homozygous form, yields pollenless plants.

14. The artichoke seed of claim 10, wherein said allele is selected from the group consisting of $ms_2$ and $ms_3$.

15. An artichoke plant male parent containing a recessive male sterility mutant allele in a homozygous form, said allele is selected from the group consisting of $ms_2$ and $ms_3$.

16. A heterozygous artichoke seed for commercial seed-planting resulting from crossing a female parent containing the $ms_2$ gene in a homozygous form with a male parent. said seed having a $ms_2$ gene as a heterozygote.

17. A heterozygous artichoke seed for commercial seed-planting resulting from crossing a female parent containing the $ms_3$ gene in a homozygous form with a male parent, said seed having a $ms_3$ gene as a heterozygote.

18. A heterozygous artichoke plant resulting from germinating the seed of claim 16.

19. A heterozygous artichoke plant resulting from germinating the seed of claim 18.

20. A method of producing globe artichoke seeds for commercial seed-planting, the method comprising the steps of:

   (a) crossing a female parent artichoke plant containing a recessive male sterility gene in a homozygous form with a male parent artichoke plant: and

   (b) collecting seeds developing on said female parent.

21. The method of claim 20, wherein said gene is selected from the group consisting of $ms_1$ $ms_2$ and $ms_3$.

22. The method of claim 20, wherein an artichoke head derived from a plant produced from said seed mimics a phenotype of a vegetatively propagated commercial artichoke variety.

Fig. 1a

Fig 1b

Fig. 3a

Fig. 3b

Fig. 2a

Fig. 2b

Fig. 4a

Fig. 4b

Fig. 5

Fig. 6

European Patent
Office

**EUROPEAN SEARCH REPORT**

Application Number

EP 98 10 6908

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X,P | EP 0 771 523 A (ENZA ZADEN DE ENKHUIZER ZAADHA) 7 May 1997 * claims 1,7 * | 1,6,7, 10,15 | A01H5/02 |
| D,X | PRINCIPE J. A.: "male-sterility in artichoke" December 1984 , HORTSCIENCE XP002073318 vol. 19(6) * page 864 * | 1,6,7, 10,15 | |
| X | DATABASE CABA PECAUT P.: "studies on globe artichoke" XP002073319 source: rapport d'activitivité, Station d'Amélioration des Plantes Maraichères, 1985-1986, (1987) pp 19-21 montfavet * abstract * | 1,6,7, 10,15 | |
| | | | **TECHNICAL FIELDS SEARCHED (Int.Cl.6)** |
| | | | A01H |

The present search report has been drawn up for all claims

| Place of search | Date of completion of the search | Examiner |
|---|---|---|
| MUNICH | 31 July 1998 | Pille, S |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)